# FASCICULE DE BREVET EUROPEEN

(11) **EP 0 200 620 B1**
(45) Date de publication et mention de la délivrance du brevet: **10.07.1991**
(21) Numéro de dépôt: 86400787.7
(22) Date de dépôt: 14.04.1986
(51) Int. Cl.: A61K 7/02

(54) **Composition cosmétique pour le démaquillage des yeux**
Kosmetisches Mittel für das Augenabschminken
Cosmetic composition for removing eye make-up

(30) Priorité: 23.04.1985 FR 8506125
(43) Date de publication de la demande: 10.12.1986
(73) Titulaire: L'OREAL, 75008 Paris (FR)
(72) Inventeur: Zabotto, Arlette, F-75014 Paris (FR); Contamin, Jean-Claude, F-91420 Morangis (FR)
(74) Mandataire: Peuscet, Jacques

(56) Documents cités:
- EP-A- 0 135 011
- EP-A- 0 141 732
- FR-A- 2 208 644
- FR-A- 2 508 795
- GB-A- 2 136 689
- US-A- 4 390 442
- CHEMICAL ABSTRACTS, vol. 96, no. 18, mai 1982, page 404, abrégé no. 148957v, Columbus, Ohio, US; K. KLEIN et al.: "Synergistic interactions of anionic/amphoteric surfactants in cosmetics", & DRUG COSMET. IND. 1981, 129(6), 38,40,42,76-7

## Description

La présente invention a pour objet une nouvelle composition cosmétique de nettoyage, notamment une lotion de démaquillage des yeux, dans laquelle la concentration en agent nettoyant est réduite de façon substantielle par comparaison avec les lotions connues pour le démaquillage des yeux, sans que son pouvoir nettoyant en soit pour autant diminué.

De très nombreux cosmétiques contenant entre autres des agents tensio-actifs et/ou des polymères non-ioniques et/ou anioniques ont été décrits.

On connaît par FR-A-2 208 644 un produit cosmétique pour le nettoyage des cheveux et de la peau contenant à côté de vaseline des détergents anioniques et amphotères, ainsi qu'éventuellement un polymère non-ionique.

Le document GB-A-2 136 689 concerne une composition épaisse ou un gel pour conditionner les cheveux. Cette composition comprend au moins une gomme de xanthane et soit au moins un polymère cationique et au moins un polymère amphotère ou anionique, soit au moins un polymère anionique et au moins un polymère cationique ou amphotère. Elle peut également comprendre, au moins un agent tensio-actif amphotère non-ionique, anionique ou cationique ou un mélange de ceux-ci.

Le document FR-A-2 508 795 est relatif à une composition cosmétique pour le traitement des cheveux et de la peau qui comprend au moins un polymère du type polyamide, tel que des poly-bêta-alanine.

Le document EP-A-0 135 011 concerne une composition pour laver et rincer les cheveux contenant des acides polyaldéhydocarboniques, ainsi que des tensio-actifs anioniques.

Aucun des documents analysés ci-dessus ne concerne le démaquillage des yeux et ne montre que l'on peut obtenir un produit contenant des tensio-actifs anioniques qui ne soit pas irritant pour la muqueuse des yeux tout en étant nettoyant.

Les agents nettoyants qui entrent dans la préparation des compositions pour le démaquillage des yeux sont, en règle générale, constitués par un mélange d'agents tensio-actifs. On s'efforce toutefois de limiter la concentration de la lotion démaquillante en agents tensio-actifs, car si ces derniers sont présents en une quantité trop importante, ils risquent d'entraîner une gêne oculaire, c'est-à-dire une irritation ou un picotement des yeux. Cependant, il ne faut pas que cette diminution de la quantité d'agents tensio-actifs s'accompagne d'une diminution des propriétés démaquillantes.

La société déposante a constaté, d'une façon tout-à-fait surprenante, que lorsque l'on utilise dans des compositions de nettoyage, en particulier de démaquillage pour les yeux, dont l'agent nettoyant est constitué par un mélange particulièrement efficace d'agents tensio-actifs anioniques et amphotères spécifiques, une proportion donnée d'au moins un polymère non-ionique et/ou d'un polymère anionique appartenant à des familles parfaitement définies, il était possible de réduire de façon substantielle les concentrations des deux agents tensio-actifs, sans pour autant influer sur les propriétés de la composition, notamment sur son pouvoir nettoyant.

Il a été constaté, en outre, que les compositions résultantes présentent les avantages complémentaires de fournir un bon confort cosmétique, c'est-à-dire qu'elles n' ont pas tendance à coller les cils et les paupières des utilisatrices, et de ne pas donner naissance à des troubles provoqués par l'instabilité de certains de leurs ingrédients, notamment des agents tensio-actifs employés, de manière à procurer une excellente stabilité non seulement au stockage, mais également dans de larges domaines de température.

La présente invention a donc pour objet le produit industriel nouveau que constitue une composition cosmétique nettoyante pour le démaquillage des yeux, à irritation oculaire réduite, comprenant, en solution aqueuse, un agent tensio-actif amphotère, un agent tensio-actif anionique et un polymère non-ionique et/ou anionique, caractérisée par le fait qu'elle comprend, en association ou non avec un additif compatible avec le démaquillage des yeux,
1°) de 0,05 à 3 % en poids en matière active d'au moins un agent tensio-actif amphotère pris dans le groupe constitué par :
   * les composés de formule (I) : 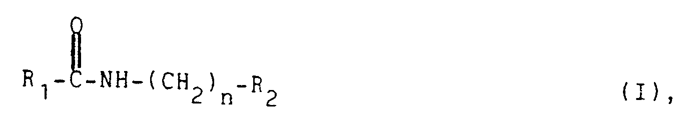 dans laquelle :
      - n est compris entre 1 et 3 ;
      - R₁ représente une chaîne grasse ayant de 7 à 17 atomes de carbone ; et
      - R₂ représente un reste de formule (II) ou (III) : 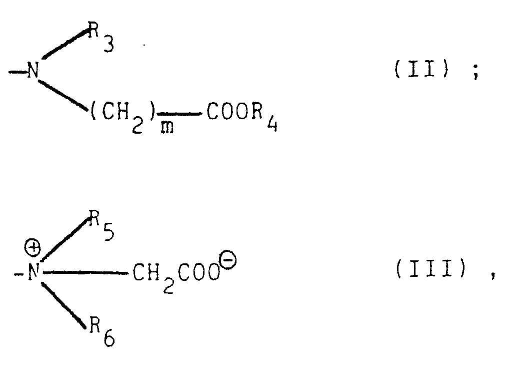 dans lesquelles :
         - m est 1 ou 2 ;
         - R₃ représente un reste -CH₂CH₂OR₄ ou un reste -CH₂CH₂OCH₂CH₂COOR₄ ;
         - R₄ représente un atome d'hydrogène ou un métal alcalin ;
         - R₅ représente un reste alkyle ou hydroxyalkyle en C₁-C₂ ; et
         - R₆ représente un reste alkyle ou hydroxyalkyle en C₁-C₂ ou un reste -(CH₂)ₘCOOR₄ , m et R₄ ayant la signification indiquée précédemment ;
   * les composés de formule (IV) : 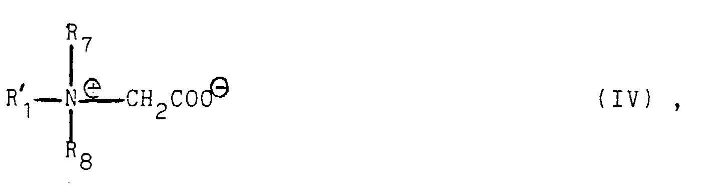 dans laquelle :
      - R'₁ représente une chaîne grasse ayant de 8 à 18 atomes de carbone ;
      - R₇ et R₈ représentent, indépendamment, un reste alkyle en C₁-C₂ ; et
   * les composés de formule (V), (VI) ou (VII) : 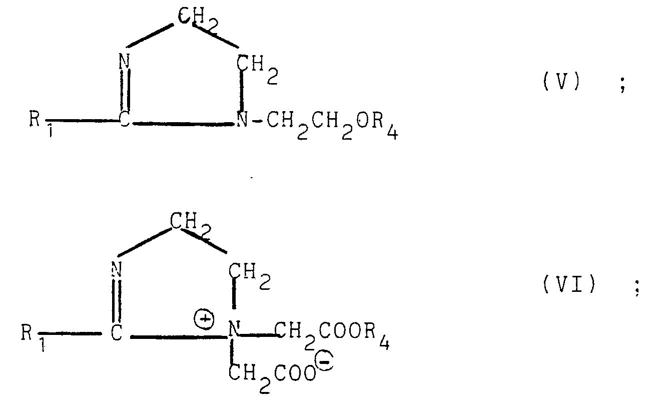 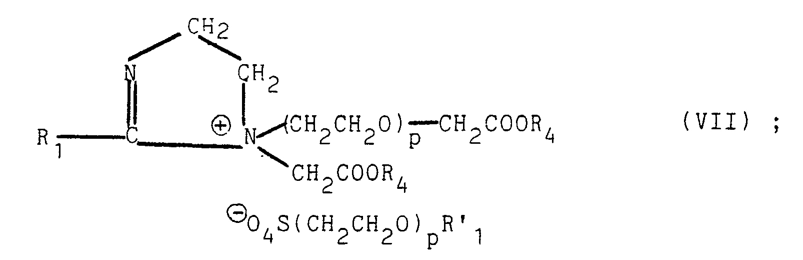 dans lesquelles
      - R₁, R'₁ et R₄ ont les significations indiquées ci-dessus ; et
      - p est compris entre 0 et 4;
2°) de 0,05 à 3% en poids en matière active d'au moins un agent tensio-actif anionique pris dans le groupe formé par les composés de formule (VIII), (IX), (X) ou (XI) : 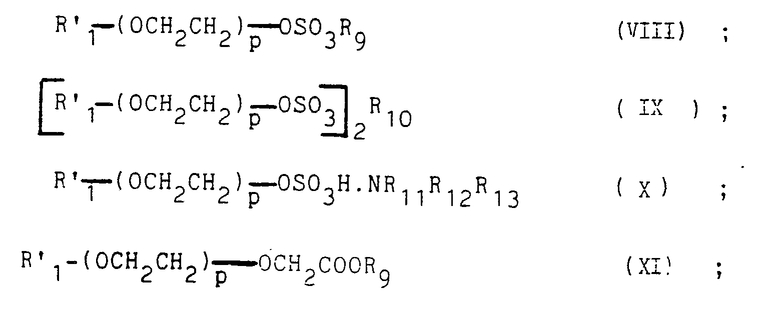 dans lesquelles :
   - R'₁ et p ont les significations indiquées précédemment ;
   - R₉ représente un métal alcalin ;
   - R₁₀ représente un métal alcalino-terreux ;
   - R₁₁, R₁₂ et R₁₃ représentent indépendamment un hydrogène, un reste alkyle en C₁-C₃ ou un reste hydroxyalkyle en C₁-C₃ ;
3°) de 0,02 à 8% en poids d'au moins un polymère non-ionique pris dans le groupe formé par les poly-β-alanines, les (C₁-C₃)-alkylcelluloses, les polyhydroxy (C₁-C₃)-alkylcelluloses, la polyvinylpyrrolidone, les dérivés non-ioniques d'amidon et les dérivés hydroxypropylés de gomme de guar, et/ou d'au moins un polymère anionique pris dans le groupe formé par les polyacrylamides contenant des groupes carboxylate, l'acide polyacrylamidosulfonique et ses sels, ainsi que les polymères et homopolymères de l'acide acrylique ou méthacrylique et leurs sels, de poids moléculaire inférieure à 100 000,

avec la condition que la concentration totale en agents tensio-actifs de la composition n'excède pas 5% en poids, les pourcentages ci-dessus étant calculés par rapport au poids total de la composition.

Conformément à un mode de réalisation préféré de la présente invention, la composition contient :
· de 0,1 à 2% en poids du (ou des) agent(s) tensio-actif(s) amphotère(s) ;
· de 0,1 à 2% en poids du (ou des) agent(s) tensio-actif(s) anionique(s) ;
· de 0,05 à 5% en poids du (ou des) polymère(s) non-ionique(s) et/ou du (ou des) polymère(s) anionique(s).

On préfère que, dans les formules (I), (V) et (VI) ci-dessus, R₁ représente une chaîne grasse en C₁₁-C₁₃, et que dans les formules (IV) et (VIII) à (XI) ci-dessus, R'₁ représente une chaîne grasse en C₁₂-C₁₄.

Parmi les agents tensio-actifs amphotères que l'on peut utiliser, on peut mentionner :
- le composé représenté par la formule : 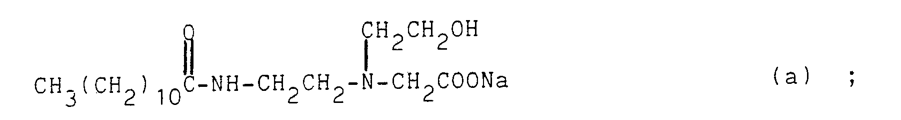 ce composé étant renfermé dans le produit commercial vendu par la société "MIRANOL" sous la désignation "MIRANOL^{®} MHT";
- les composés suivants dans lesquels le radical R signifie un reste d'acides gras du coprah : 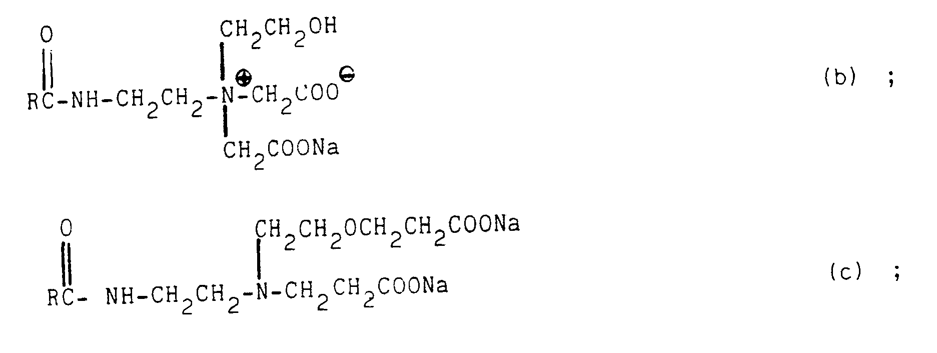 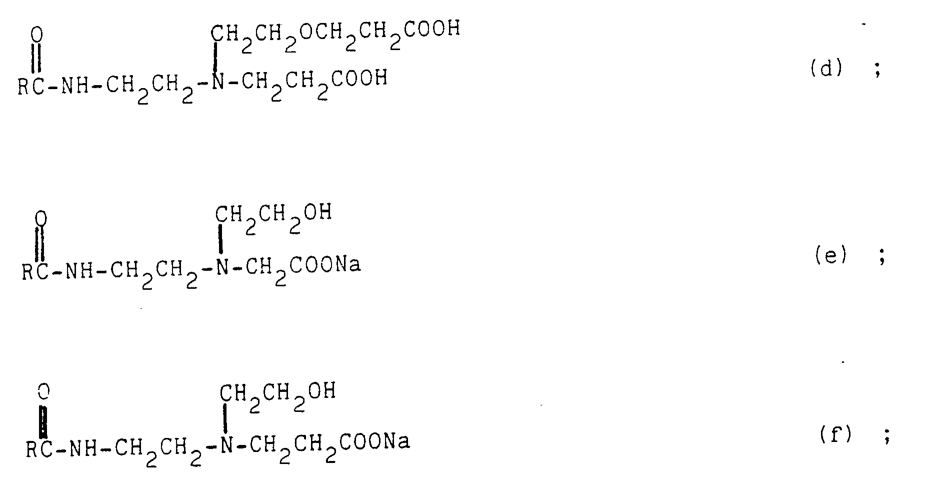 vendus par la Société "MIRANOL" sous les désignations respectivement de :
   - "MIRANOL C2M Conc. N.P." (ou "MIRANOL C2M Conc. O.P." ; ou encore "MIRANOL CM Special") ;
   - "MIRANOL C2M-SF 70%, C2M-SF 75%, C2M-SF Conc., C2M-SFE Conc. ou C2M-SFP" ;
   - "MIRANOL C2M Anhydrous acid" ;
   - "MIRANOL CM Conc. N.P." ; et
   - "MIRANOL CM-SF Conc." (ou "MIRANOL CM-SFX Conc.").
- le composé suivant dans lequel R signifie un reste d'acides gras de coprah : 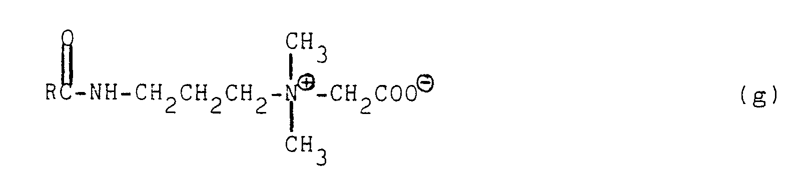 vendu par la Société "HENKEL" sous la désignation "DEHYTON^{®} K" ;
- le composé de formule : 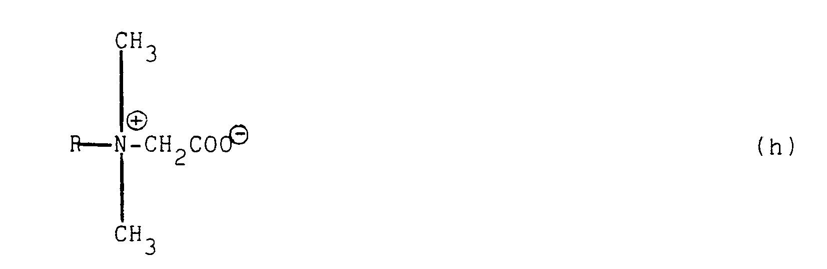 vendu par la Société "HENKEL" sous la désignation "DEHYTON AB-30".
- le composé suivant dans lequel R signifie un reste d'acides gras du coprah : 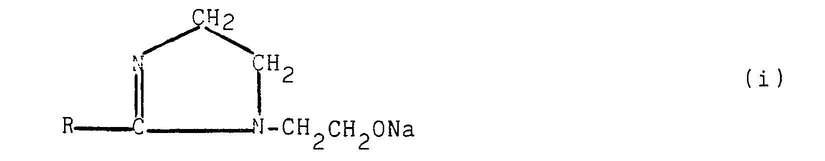 vendu par la Société "HENKEL" sous la désignation "DEHYTON G".
- le composé suivant dans lequel R signifie un reste d'acides gras du coprah : 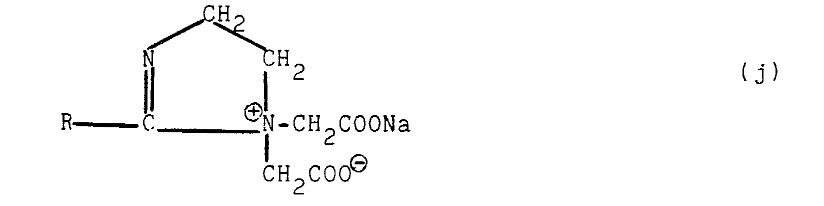 vendu par la Société "NAARDEN" sous la désignation "AMPHOLAK^{®} XCO-30".
- le composé représenté par la formule : 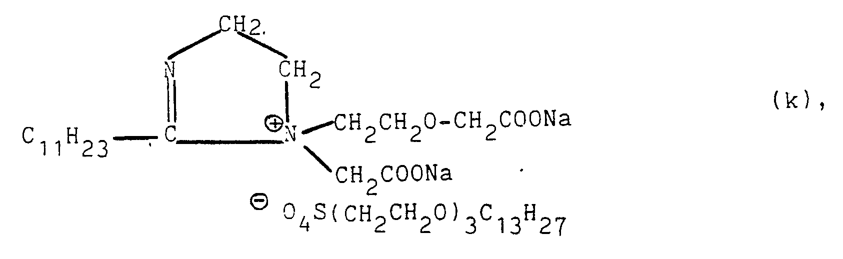 ce composé étant renfermé dans le produit commercial vendu par la Société "MIRANOL" sous la désignation "MIRANOL 2MHT modified".

Parmi les agents tensio-actifs anioniques, on peut, en particulier, mentionner ceux qui sont représentés par les formules suivantes, dans lesquelles n est compris entre 1 et 4 :

CH₃(CH₂)₁₀CH₂OSO₃NH₄ (l) ;

[CH₃(CH₂)₁₀CH₂(OCH₂CH₂)ₙOSO₃]₂Mg (m)

;

CH₃(CH₂)₁₀CH₂OSO₃H.H₂NCH₂CH₂OH (n)

;

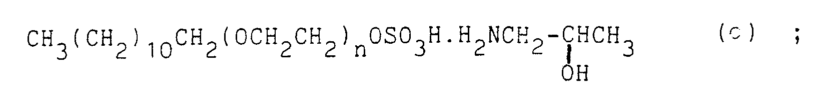

CH₃(CH₂)₁₀CH₂OSO₃Na (p) ;

CH₃(CH₂)₁₂CH₂(OCH₂CH₂)ₙOSO₃Na (q)

;

CH₃(CH₂)₁₀CH₂(OCH₂CH₂)ₙOSO₃H.N (CH₂CH₂OH)₃ (r) ;

CH₃(CH₂)₁₀CH₂(OCH₂CH₂)ₙOCH₂ COONa (s) ;

qui sont commercialisés par la Société "HENKEL" respectivement sous les désignations :
- "TEXAPON® A-400" ;
- "TEXAPON MG" ;
- "TEXAPON MLS" ;
- "TEXAPON IES"(ou "TEXAPON WW99") ;
- "TEXAPON K12" (ou K-1296, L-100, V HC Needles, ZHC Needles, ZHC Powder") ;
- "TEXAPON K14S Special" ;
- "TEXAPON NT" ; et

par la Société "SANDOZ" sous la désignation "SANDOPAN® LS-24".

On peut également mentionner, comme agent tensio-actif anionique, le composé de formule :

C₁₃H₂₇(OCH₂CH₂)ₙOSO₃Na (t)

;

dans laquelle n = 1 à 4,
ce composé étant renfermé dans les produits commerciaux vendus par la société "MIRANOL" sous les désignations "MIRANOL 2MCT Modified, 2MHT Modified , BT Modified ou MHT".

Parmi les polymères non-ioniques, qui peuvent être utilisés dans les compositions selon l'invention, on peut en particulier citer :
1°) les poly-bêta-alanines décrites dans le brevet belge n^{o} 893 738.

Ces polymères comportent de 50 à 100 % de motifs répétitifs du type β-alanine correspondant à la formule suivante : 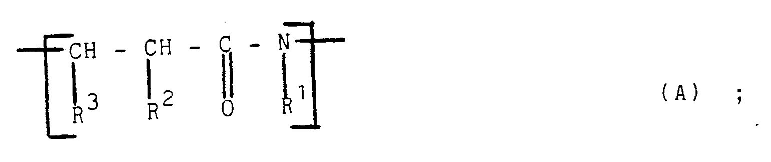 et de 0 à 50% de motifs répétitifs du type acrylamide correspondant à la formule suivante : 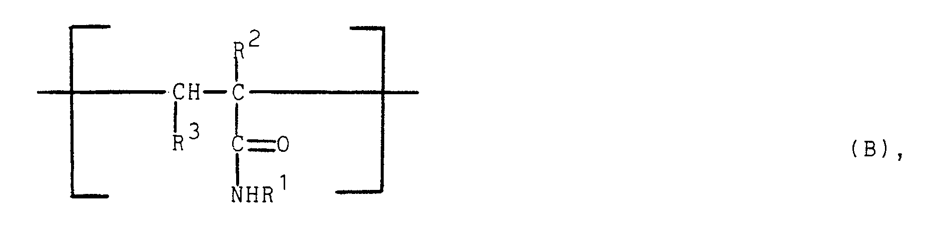 dans lesquelles :
- R¹ représente un atome d'hydrogène ou un radical pris dans le groupe constitué par les radicaux suivants: 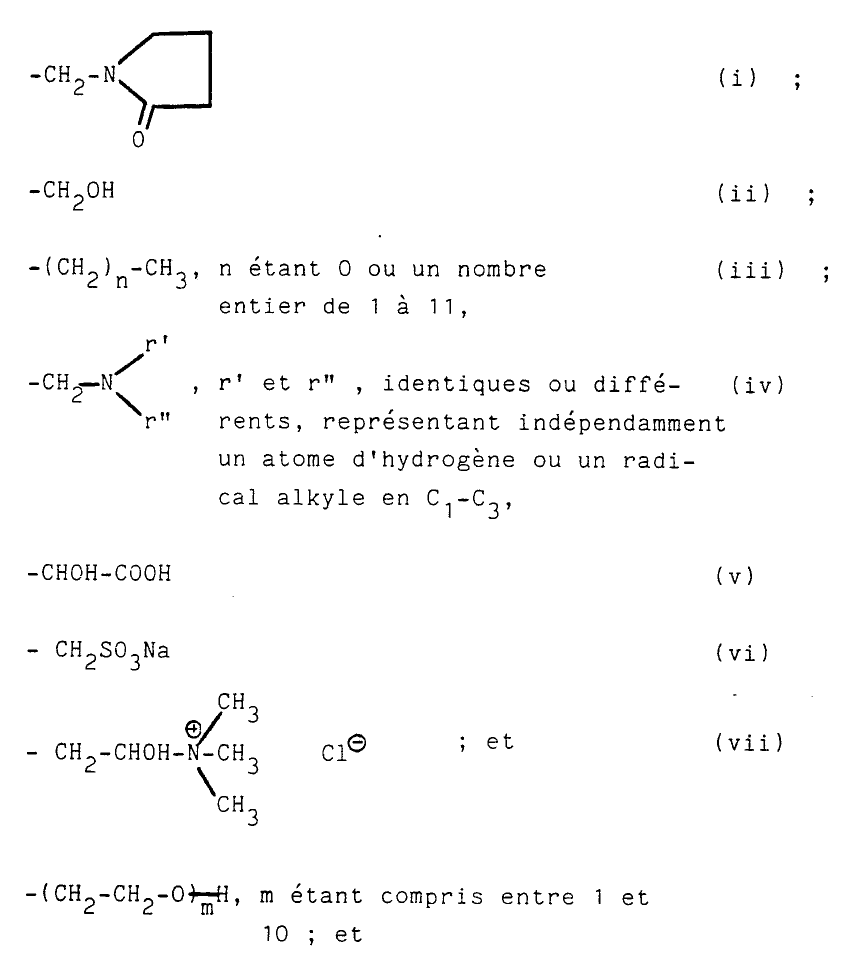
- R² et R³ représentent un atome d'hydrogène ou un radical méthyle.

Ces polymères sont préparés par polymérisation de l'acrylamide, comme décrit dans le brevet des Etats-Unis d'Amérique n^{o} 4 082 730. Ils ont, de préférence, un poids moléculaire compris entre 500 et 200 000 et, plus particulièrement, entre 2 000 et 100 000.
2°) la polyvinylpyrrolidone de poids moléculaire d'environ 40 000, telle que celle vendue par la société "GAF" sous la désignation "PVP K-30" ;
3°) l'hydroxyéthylcellulose, comme par exemple la substance vendue sous la dénomination "NATROSOL® 250" par la Société "HERCULES".
4°) les dérivés hydroxypropylés de gomme de guar, en particulier le produit vendu par la Société "MEYHALL" sous la désignation "JAGUAR® HP-8".

Parmi les polymères anioniques qui peuvent être utilisés dans les compositions selon l'invention, on peut en particulier citer :
1°/ les polyacrylamides contenant des groupes carboxylate tels que ceux vendus par la Société "AMERICAN CYANAMID" sous la désignation "CYANAMER® A 370" ;
2°/ les sels d'acide polyacrylamide-sulfonique tels que ceux mentionnés dans le brevet américain n^{o} 4 128 631, et plus particulièrement ceux de l'acide polyacrylamidométhylpropane-sulfonique, tel que celui vendu par la Société "HENKEL" sous la désignation "COSMEDIA® POLYMER HSP 1180" et constitué par une chaîne alkyle linéaire avec des motifs pendants qui sont des groupes acide amidométhylpropane-sulfonique, comme représenté ci-après : 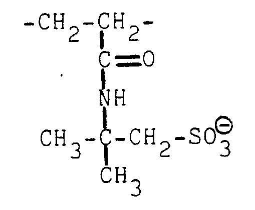
3°/ le polyméthacrylate de sodium, tel que celui vendu par la Société "VANDERBILT" sous la désignation "DARVAN® n^{o} 7", et
4°/ les polymères d'acide acrylique tel que celui vendu par la Société "ALLIED COLLOIDS" sous la désignation "VERSICOL® E5" et ayant un poids moléculaire d'environ 3500.

Le véhicule des compositions de nettoyage selon l'invention est, soit de l'eau déminéralisée stérile, soit une eau "florale", telle que de l'eau de rose, de l'eau de bleuet, de l'eau de camomille, ou de l'eau de tilleul.

Les autres ingrédients de la composition de nettoyage selon l'invention sont essentiellement un agent conservateur, qui peut être, par exemple, de l'éthylmercurithiosalicylate de sodium, un sel de chlorhexidine tel que le digluconate, le diacétate et le dichlorhydrate, un sel de phénylmercure tel que le nitrate de phénylmercure, un mélange constitué de 30% en poids de benzoate de sodium et de 70% en poids de monochloracétamide, un composé de formule (XII) : 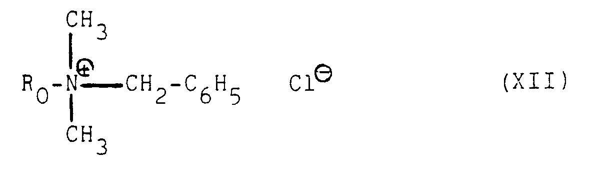 dans laquelle :
- R₀ représente un radical alkyle en C₁₂-C₁₈ ou un mélange de tels radicaux alkyle tels que les mélanges C₁₂-C₁₄ et C₁₄-C₁₆.

L'agent conservateur des compositions selon l'invention est généralement à une concentration comprise entre 0,002 et 0,8% en poids, et, de préférence, entre 0,02 et 0,5% en poids.

Le pH des compositions selon l'invention est généralement compris entre 7 et 8,5 et, de préférence, entre 7 et 8 ; il est obtenu à l'aide d'un agent tampon tel que, par exemple, un tampon phosphate (hydrogénophosphate de dipotassium/dihydrogénophosphate de potassium).

Les compositions selon l'invention peuvent également contenir d'autres adjuvants conventionnels tels que, par exemple, des agents humectants, des agents adoucissants, des parfums ou des colorants, ceux-ci devant, bien entendu, présenter la particularité d'être stables au sein de la composition et ne pas provoquer d'irritation ou de picotements de la muqueuse oculaire.

Parmi les agents humectants, on peut, en particulier, citer le méthyl-2 pentanediol-2,4 et le polyéthylèneglycol d'un poids moléculaire d'environ 600.

Parmi les agents adoucissants, on peut en particulier mentionner l'allantoïne et l'azulène.

Pour mieux faire comprendre l'objet de l'invention, on va en décrire maintenant, à titre d'exemples purement illustratifs et non limitatifs, plusieurs modes de mise en oeuvre.

### EXEMPLE 1

On prépare une lotion démaquillante pour les yeux formulée comme suit :

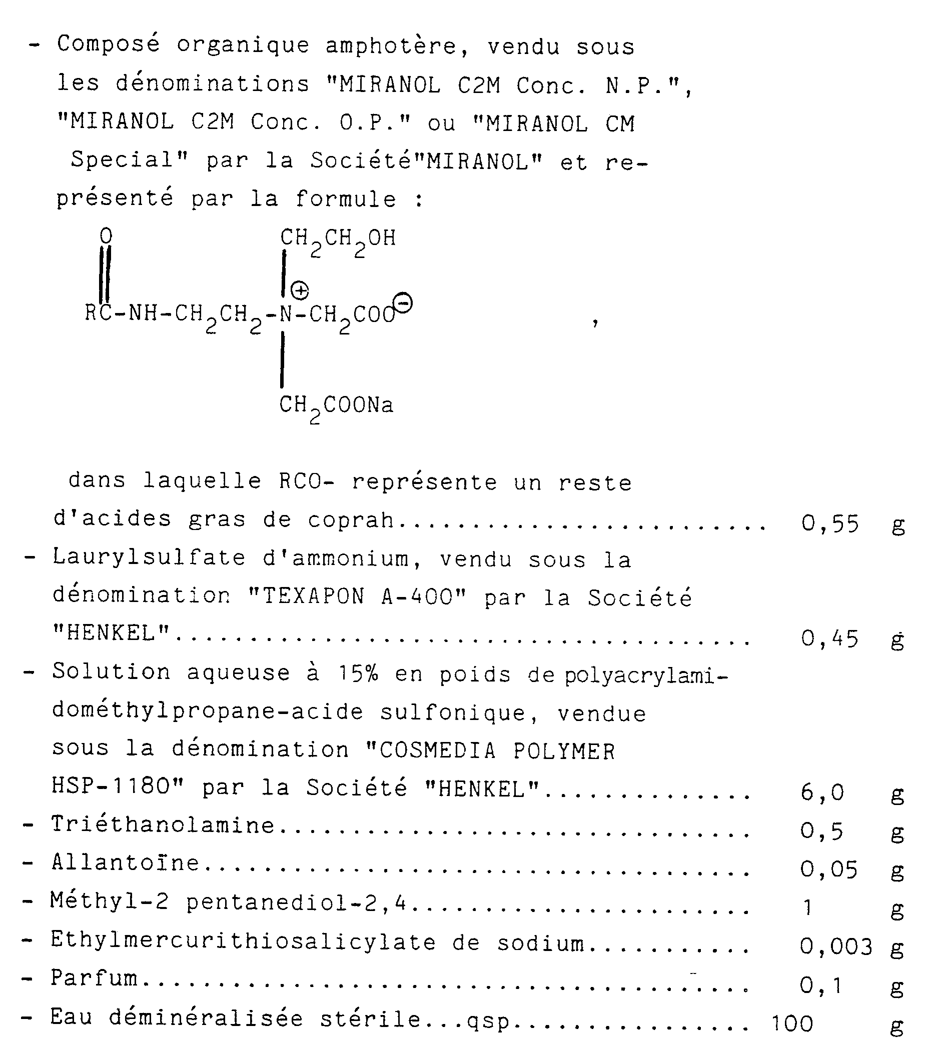

Cette lotion présente un pH de 8 environ.

Cette lotion, appliquée le soir avant le coucher, permet un démaquillage rapide sans irriter le contour des yeux.

### EXEMPLE 2

On prépare une lotion démaquillante pour les yeux formulée comme suit :

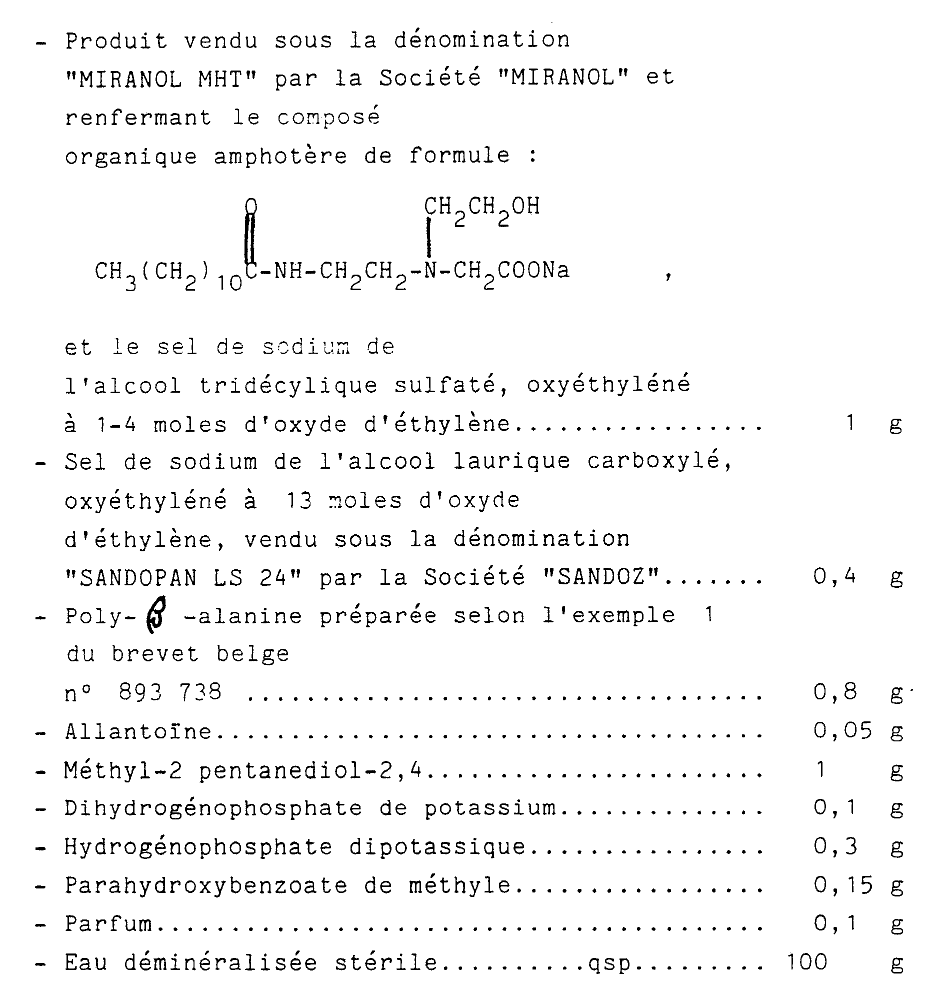

Cette lotion présente un pH de 7,3.

Cette lotion est particulièrement bien adaptée pour démaquiller les yeux sans avoir tendance à coller les cils et les paupières des utilisatrices.

### EXEMPLE 3

On prépare une lotion démaquillante pour les yeux formulée comme suit :

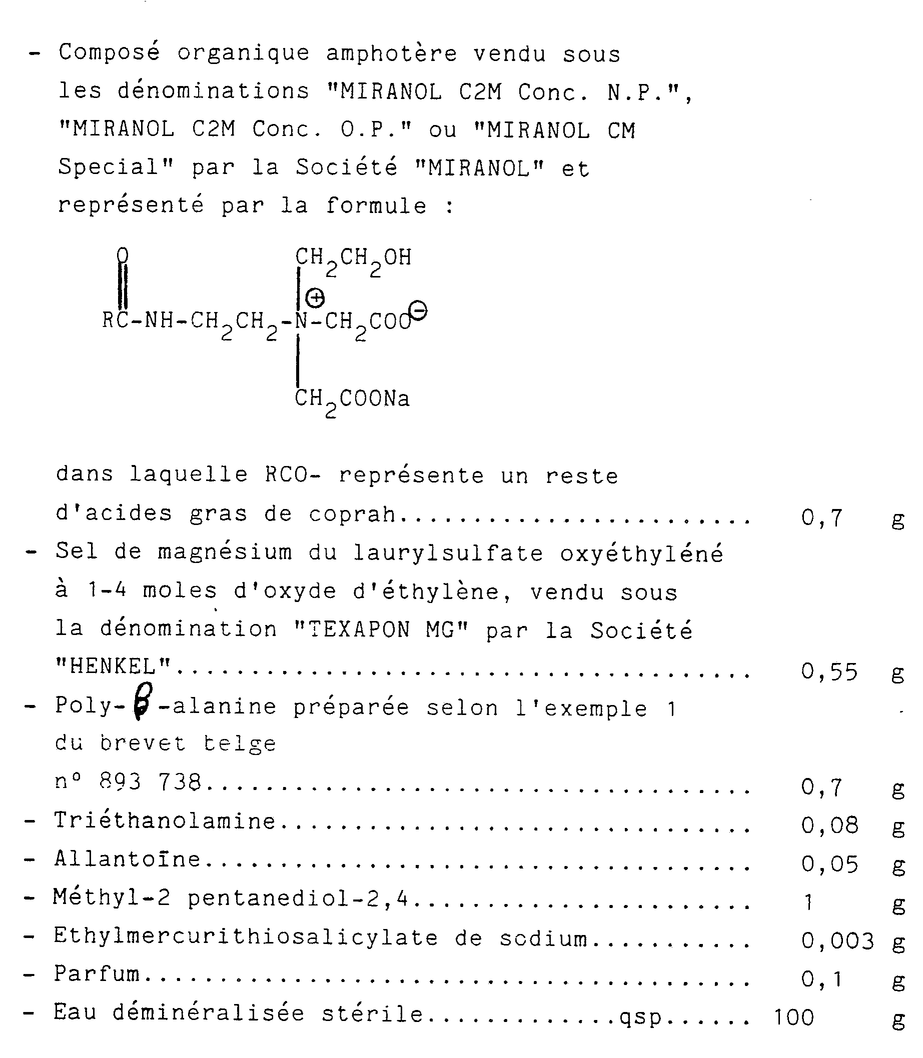

Cette lotion présente un pH de 8 environ.

Elle démaquille bien tout en apportant un bon confort à l'utilisatrice.

### EXEMPLE 4

On prépare une lotion démaquillante pour les yeux formulée comme suit :

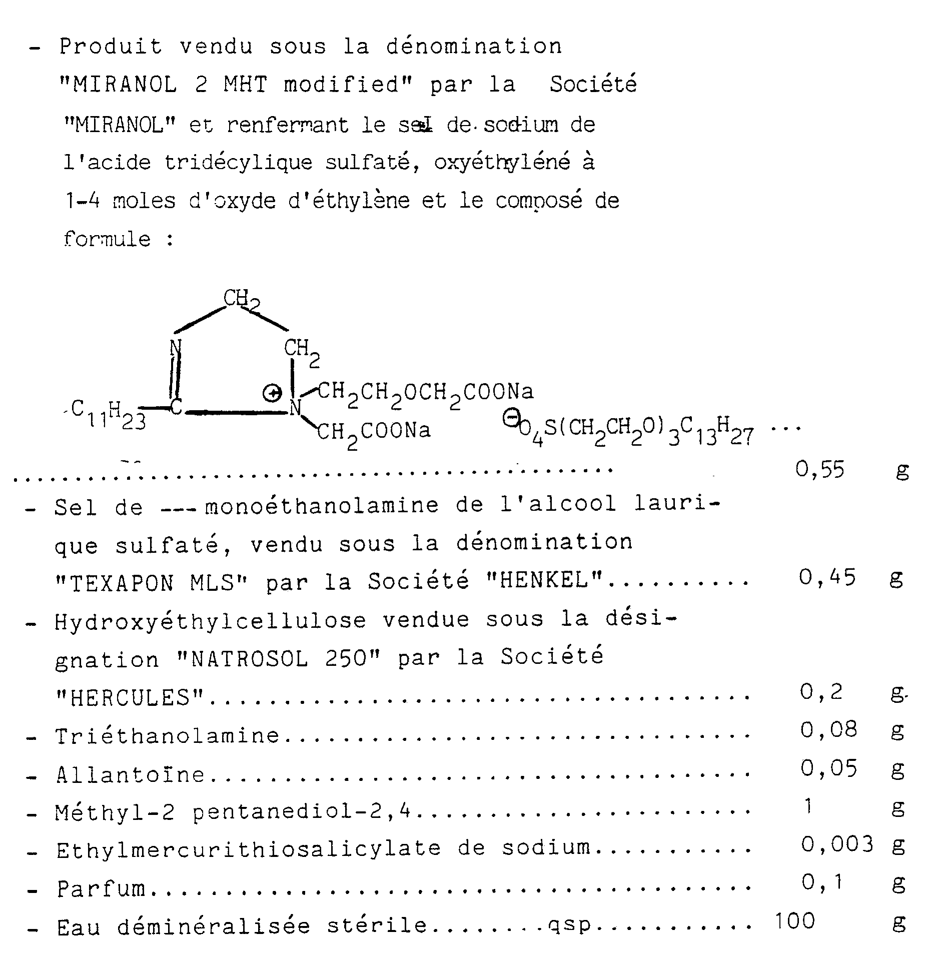

Cette lotion présente un pH d'environ 8.

Cette lotion, utilisée le soir, élimine parfaitement le maquillage sans provoquer une irritation des paupières.

### EXEMPLE 5

On prépare une lotion démaquillante pour les yeux formulée comme suit :

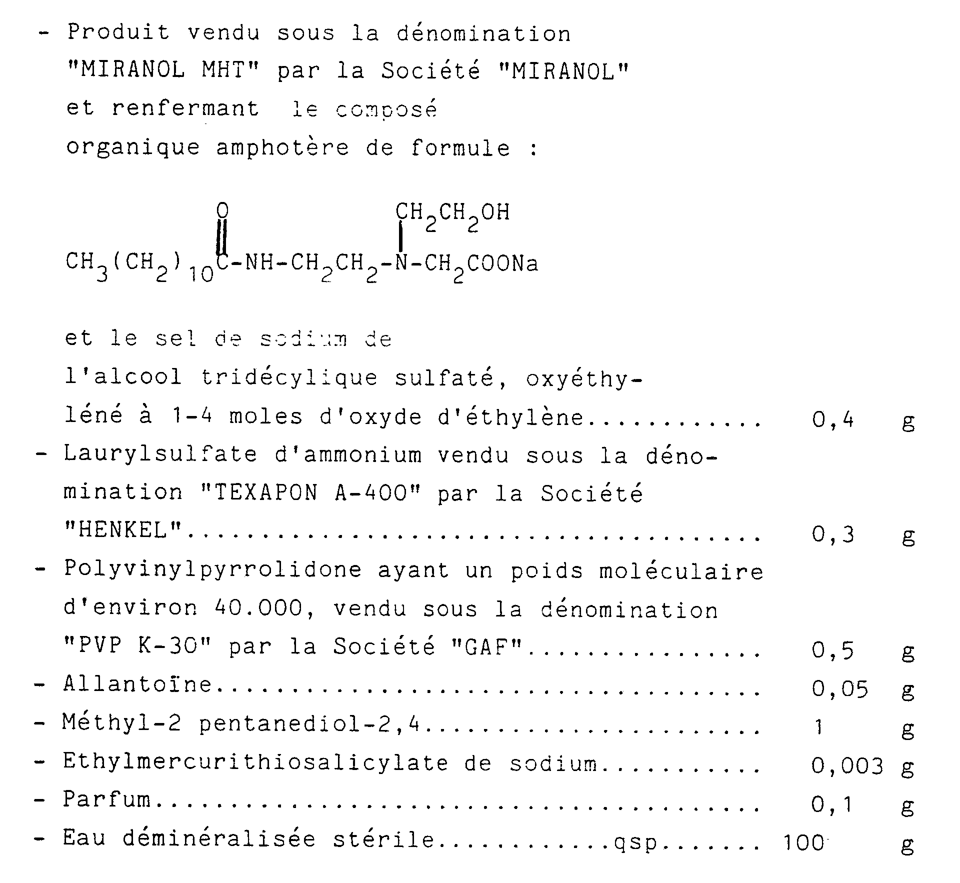

Cette lotion présente un pH de 7,8.

Cette lotion permet un démaquillage rapide et très efficace sans irriter les yeux.

## Revendications

1. Composition cosmétique nettoyante pour le démaquillage des yeux, à irritation oculaire réduite, comprenant, en solution aqueuse, un agent tensio-actif amphotère, un agent tensio-actif anionique et un polymère non-ionique et/ou anionique, caractérisée par le fait qu'elle comprend, en association ou non avec un additif compatible avec le démaquillage des yeux,
1°) de 0,05 à 3% en poids en matière active d'au moins un agent tensio-actif amphotère pris dans le groupe constitué par :
* les composés de formule (I) : 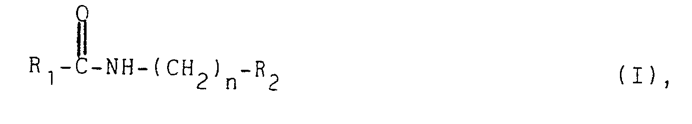 dans laquelle :
- n est compris entre 1 et 3 ;
- R₁ représente une chaîne grasse ayant de 7 à 17 atomes de carbone ; et
- R₂ représente un reste de formule (II) ou (III) : 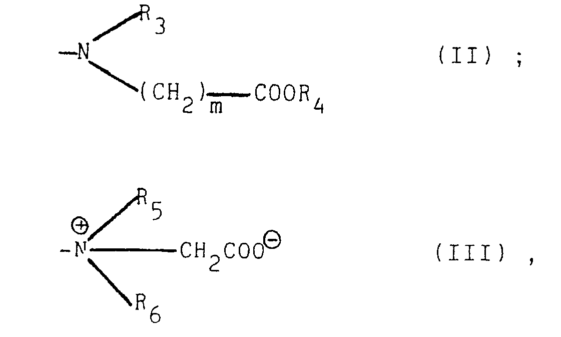 dans lesquelles :
- m est 1 ou 2 ;
- R₃ représente un reste -CH₂CH₂OR₄ ou un reste -CH₂CH₂OCH₂CH₂COOR₄ ;
- R₄ représente un atome d'hydrogène ou un métal alcalin ;
- R₅ représente un reste alkyle ou hydroxyalkyle en C₁-C₂ ; et
- R₆ représente un reste alkyle ou hydroxyalkyle en C₁-C₂ ou un reste -(CH₂)ₘCOOR₄ , m et R₄ ayant la signification indiquée précédemment ;
* les composés de formule (IV) : 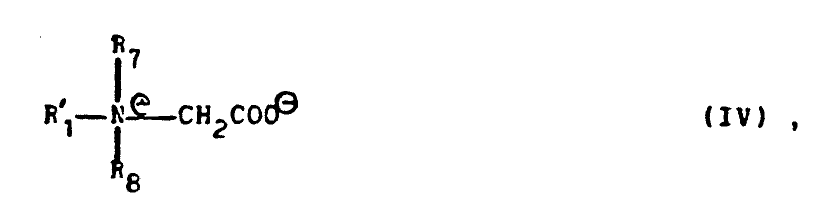 dans laquelle :
- R'₁ représente une chaîne grasse ayant de 8 à 18 atomes de carbone ;
- R₇ et R₈ représentent, indépendamment, un reste alkyle en C₁-C₂ ; et
* les composés de formule (V), (VI) ou (VII) : 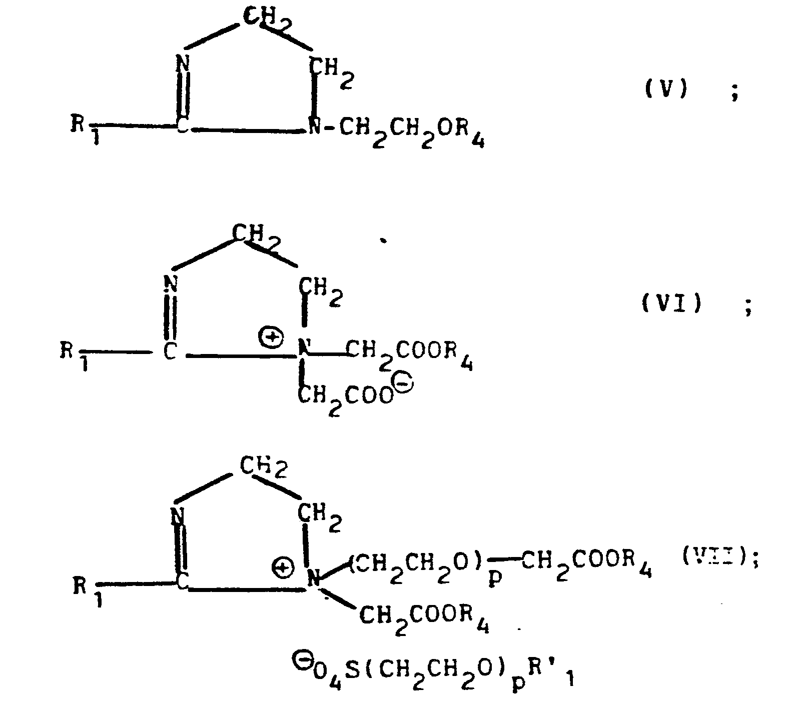 dans lesquelles
- R₁, R'₁ et R₄ ont les significations indiquées ci-dessus ; et
- p est compris entre 0 et 4;
2°) de 0,05 à 3% en poids en matière active d'au moins un agent tensio-actif anionique pris dans le groupe formé par les composés de formule (VIII), (IX), (X) ou (XI) : 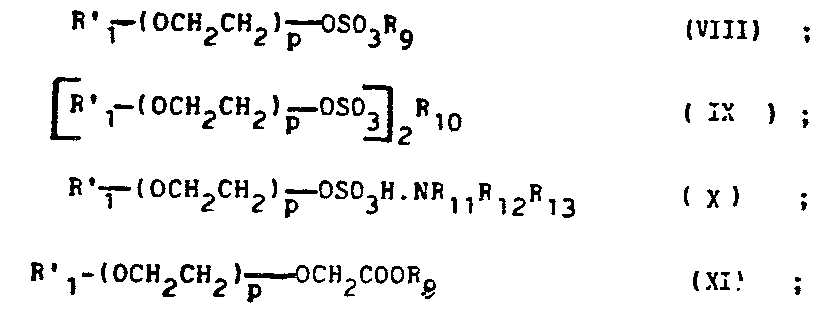 dans lesquelles :
- R'₁ et p ont les significations indiquées précédemment ;
- R₉ représente un métal alcalin ;
- R₁₀ représente un métal alcalino-terreux ;
- R₁₁, R₁₂ et R₁₃ représentent indépendamment un hydrogène, un reste alkyle en C₁-C₃ ou un reste hydroxyalkyle en C₁-C₃ ;
3°) de 0,02 à 8% en poids d'au moins un polymère non-ionique pris dans le groupe formé par les poly-β-alanines, les (C₁-C₃)-alkylcelluloses, les polyhydroxy(C₁-C₃)-alkyl-celluloses, la polyvinylpyrrolidone, les dérivés non-ioniques d'amidon et les dérivés hydroxypropylés de gomme de guar, et/ou d'au moins un polymère anionique pris dans le groupe formé par les polyacrylamides contenant des groupes carboxylate, l'acide polyacrylamidosulfonique et ses sels, ainsi que les polymères et homopolymères de l'acide acrylique ou méthacrylique et leurs sels, de poids moléculaire inférieure à 100 000,
avec la condition que la concentration totale en agents tensio-actifs de la composition n'excède pas 5% en poids, les pourcentages ci-dessus étant calculés par rapport au poids total de la composition.

2. Composition selon la revendication 1, caractérisée par le fait qu'elle contient :
· de 0,1 à 2% en poids du (ou des) agent(s) tensio-actif(s) amphotère(s) ;
· de 0,1 à 2% en poids du (ou des) agent(s) tensio-actif(s) anionique(s) ;
· de 0,05 à 5% en poids du (ou des) polymère(s) non-ionique(s) et/ou du (ou des) polymère(s) anionique(s).

3. Composition selon l'une des revendications 1 et 2, caractérisée par le fait que, dans les formules (I), (V) et (VI) indiquées à la revendication 1, R₁ représente une chaîne grasse en C₁₁-C₁₃, et dans les formules (IV) et (VIII) à (XI) également indiquées à la revendication 1, R'₁ représente une chaîne grasse en C₁₂-C₁₄.

4. Composition selon l'une des revendications 1 à 3, caractérisée par le fait que le (ou les) agent(s) tensio-actif(s) amphotère(s) est (ou sont) pris dans le groupe constitué par les composés ayant les formules suivantes : 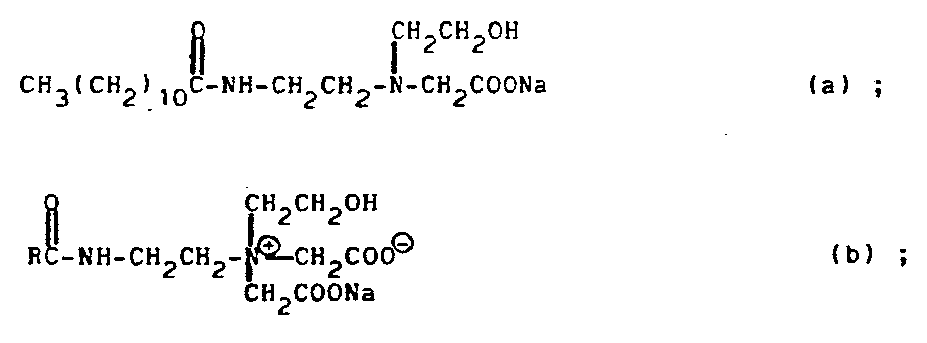 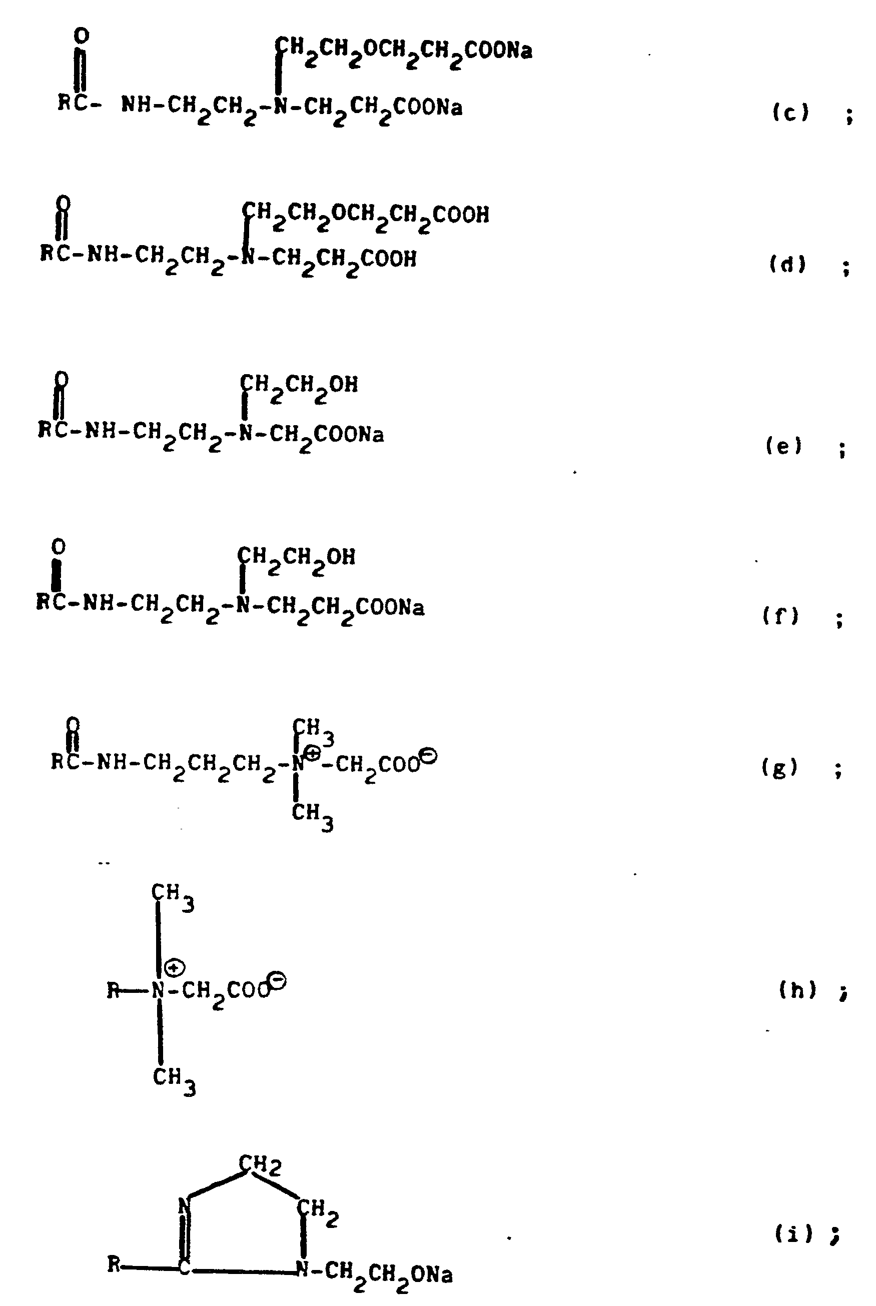 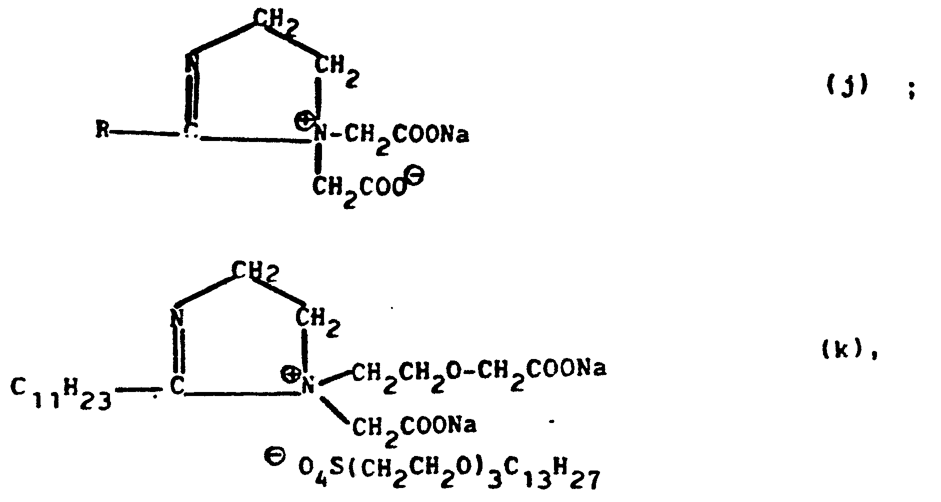 R, dans les formules (b) à (j), représentant un reste d'acides gras de coprah.

5. Composition selon l'une des revendications 1 à 4, caractérisée par le fait que le (ou les) agent(s) tensio-actif(s) anionique(s) est (ou sont) pris dans le groupe constitué par les composés ayant les formules suivantes :
CH₃(CH₂)₁₀CH₂OSO₃NH₄ (l) ;
[CH₃(CH₂)₁₀CH₂(OCH₂CH₂)ₙOSO₃]₂Mg (m) ;
CH₃(CH₂)₁₀CH₂OSO₃H.H₂NCH₂CH₂OH (n) ; 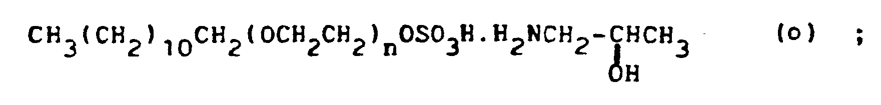
CH₃(CH₂)₁₀CH₂OSO₃Na (p) ;
CH₃(CH₂)₁₂CH₂(OCH₂CH₂)ₙOSO₃Na (q) ;
CH₃(CH₂)₁₀CH₂(OCH₂CH₂)ₙOSO₃H.N (CH₂CH₂OH)₃ (r) ;
CH₃(CH₂)₁₀CH₂(OCH₂CH₂)ₙO CH₂COONa (s) ;
C₁₃H₂₇(OCH₂CH₂)ₙOSO₃Na (t) ;
n, dans les formules (m) à (t), étant compris entre 1 et 4.

6. Composition selon l'une des revendications 1 à 5, caractérisée par le fait qu'elle présente un pH compris entre 7 et 8,5.

7. Composition selon la revendication 6, caractérisée par le fait qu'elle présente un pH compris entre 7 et 8.

8. Composition selon l'une des revendications 1 à 7, caractérisée par le fait qu'elle contient au moins un agent conservateur pris dans le groupe constitué par l'éthylmercurithiosalicylate de sodium, un sel de chlorhexidine tel que le digluconate, le diacétate et le dichlorhydrate, un sel de phénylmercure tel que le nitrate de phénylmercure, un mélange constitué de 30% en poids de benzoate de sodium et de 70% en poids de monochloracétamide, un composé de formule (XII): 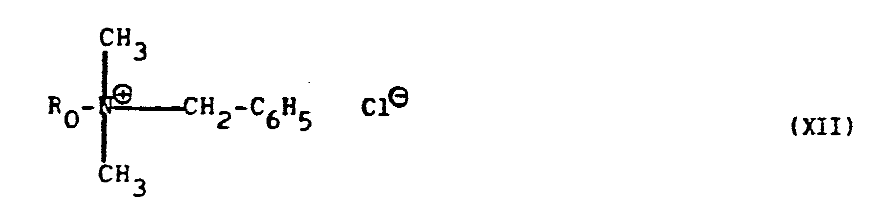 dans laquelle :
- R₀ représente un radical alkyle en C₁₂-C₁₈ ou un mélange de tels radicaux alkyle ainsi que les mélanges C₁₂-C₁₄ et C₁₄-C₁₆.

9. Composition selon la revendication 8, caractérisée par le fait que la concentration en agent(s) conservateur(s) est comprise entre 0,002 et 0,8% en poids, de préférence entre 0,02 et 0,5% en poids, par rapport au poids total de la composition.

10. Composition selon l'une des revendications 1 à 9, caractérisée par le fait qu'elle contient au moins un additif pris dans le groupe formé par les agents humectants, les agents adoucissants, les parfums et les colorants.

## Claims

1. Cosmetic cleansing composition for removing eye make-up, with reduced eye irritation, comprising, in an aqueous solution, an amphoteric surface-active agent, an anionic surface-active agent and a nonionic and/or anionic polymer characterised in that it comprises, whether or not in combination with an additive which is compatible with removing eye make-up,
1) from 0.05 to 3% by weight, as active substance of at least one amphoteric surface-active agent taken from the group consisting of:
- the compounds of formula (I): 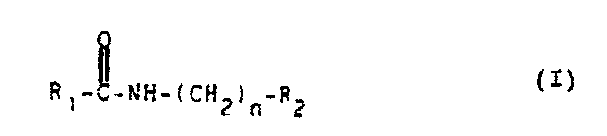 in which:
- n is between 1 and 3;
- R₁ denotes a fatty chain containing from 7 to 17 carbon atoms; and
- R₂ denotes a residue of formula (II) or (III): 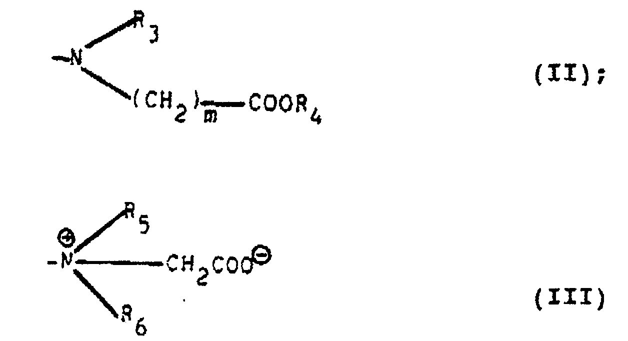 in which:
- m is 1 or 2;
- R₃ denotes a residue - CH₂CH₂OR₄ or a residue -CH₂CH₂OCH₂CH₂COOR₄;
- R₄ denotes a hydrogen atom or an alkali metal;
- R₅ denotes a C₁-C₂ alkyl or hydroxyalkyl residue; and
- R₈ denotes a C₁-C₂ alkyl or hydroxyalkyl residue or a residue -(CH₂)ₘCOOR₄, m and R₄, having the meaning given above;
- the compounds of formula (IV): 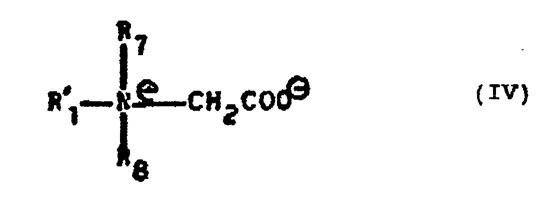 in which:
- R'₁ denotes a fatty chain containing from 8 to 18 carbon atoms;
- R₇ and R₈ denote, independently, a C₁-C₂ alkyl residue; and
- the compounds of formula (V), (VI) or (VII): 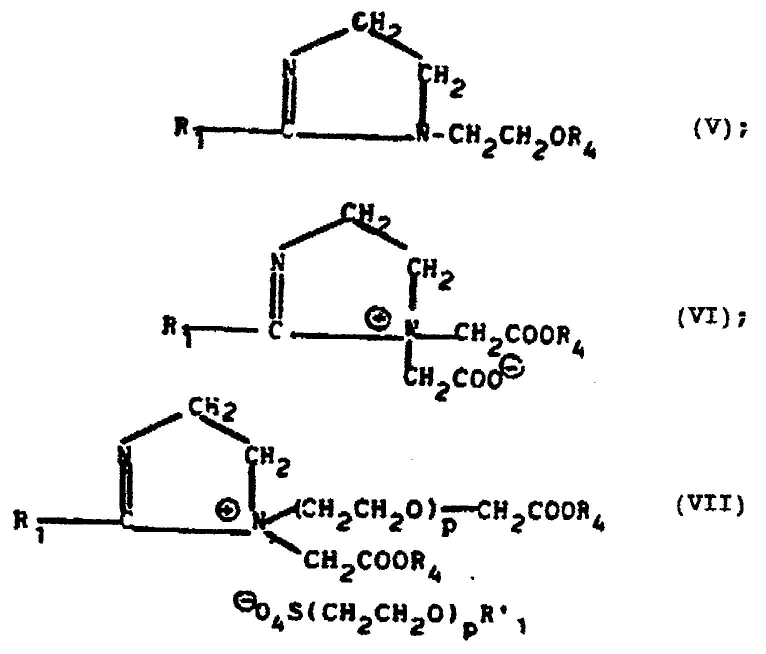 in which
- R₁, R'₁ and R₄ have the meanings given above; and
- p is from 0 to 4;
2) from 0.05 to 3% by weight as active material of at least one anionic surface-active agent taken from the group consisting of the compounds of formula (VIII), (IX), (X) or (XI): 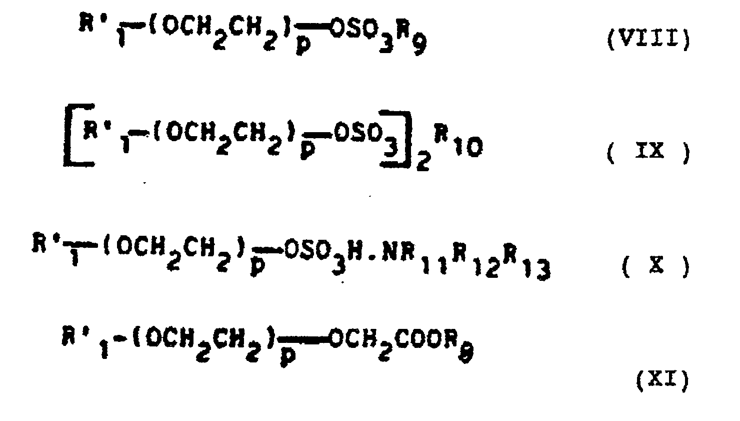 in which:
- R'₁ and p have the meanings given above;
- R₉ denotes an alkali metal;
- R₁₀ denotes an alkaline-earth metal;
- R₁₁, R₁₂ and R₁₃ independently denote a hydrogen, a C₁-C₃ alkyl residue or a C₁-C₃ hydroxyalkyl residue;
3) from 0.02 to 8% by weight of at least one nonionic polymer taken from the group consisting of poly-β-alanines, C₁-C₃-alkyl celluloses, polyhydroxy-(C₁-C₃-alkyl) celluloses, polyvinylpyrrolidone, nonionic starch derivatives and hydroxypropylated derivatives of guar gum, and/or at least one anionic polymer taken from the group consisting of polyacrylamides containing carboxylate groups, polyacrylamidosulphonic acid and its salts, and the polymers and homopolymers of acrylic or methacrylic acid and their salts, with a molecular weight of less than 100,000,
on condition that the total concentration of the surface-active agents in the composition does not exceed 5% by weight, the above percentages being calculated relative to the total weight of the composition.

2. Composition according to Claim 1, characterised in that it contains:
- from 0.1 to 2% by weight of the amphoteric surface-active agent(s);
- from 0.1 to 2% by weight of the anionic surface-active agent(s);
- from 0.05 to 5% by weight of the nonionic polymer(s) and/or of the anionic polymer(s).

3. Composition according to either of Claims 1 and 2, characterised in that, in the formulae (I), (V) and (VI) indicated in Claim 1, R₁ denotes a C₁₁-C₁₃ fatty chain, and in the formulae (IV) and (VIII) to (XI), also indicated in Claim 1, R'₁ denotes a C₁₂-C₁₄ fatty chain.

4. Composition according to one of Claims 1 to 3, characterised in that the amphoteric surface-active agent(s) is (or are) taken from the group consisting of the compounds having the following formulae: 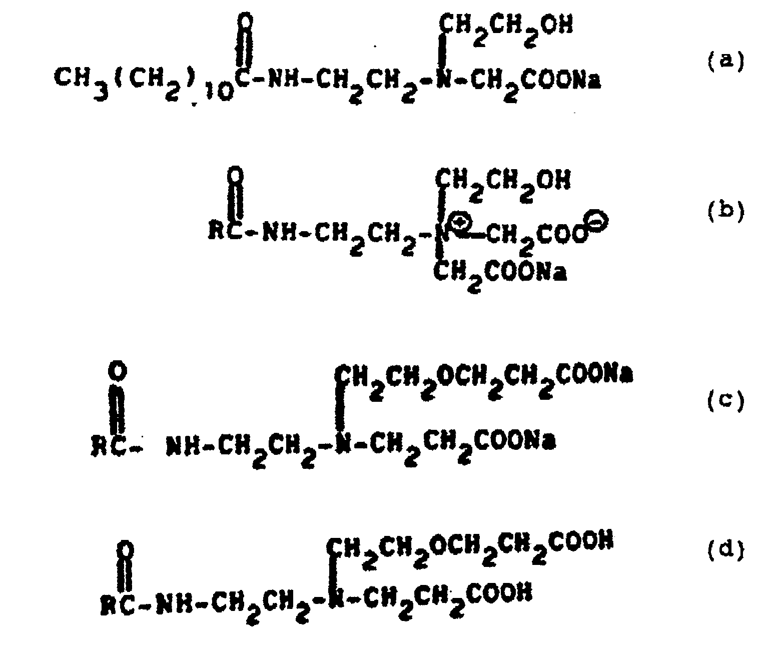 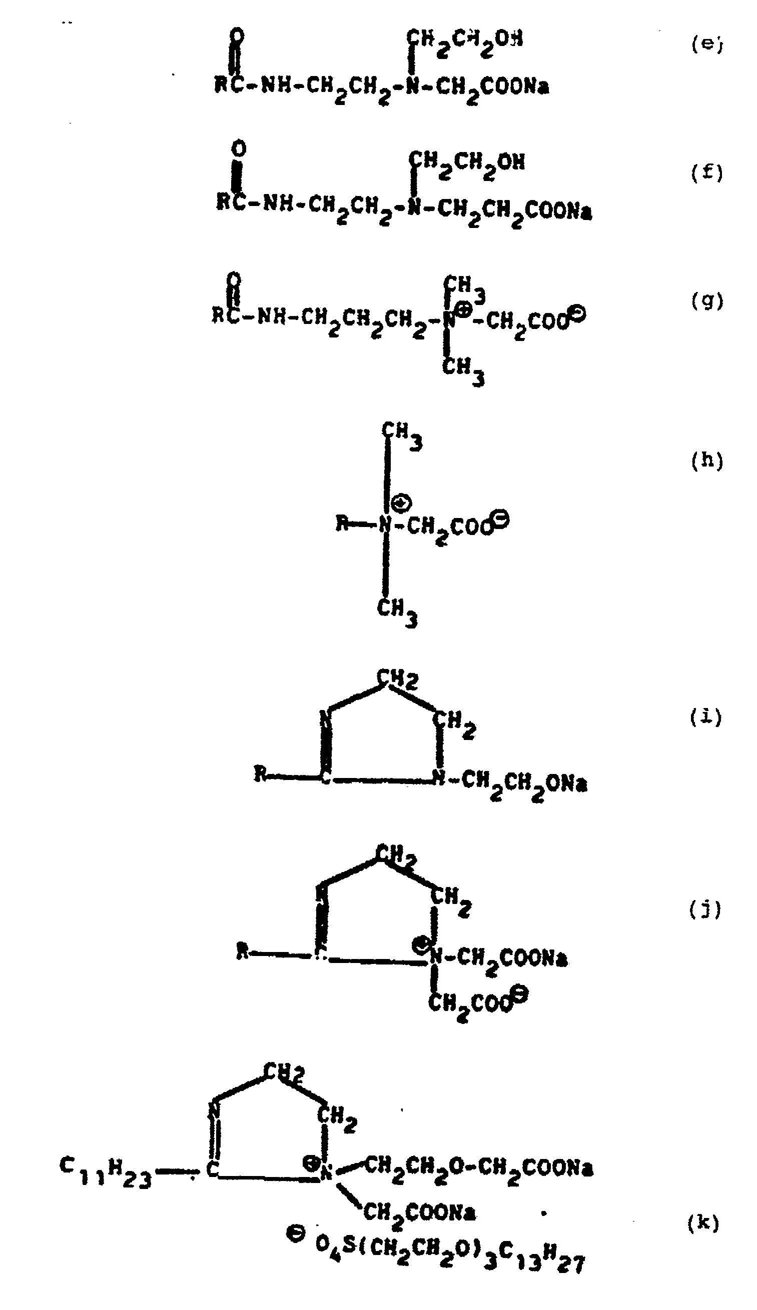 R, in the formula (b) to (j), denoting a residue of coprah fatty acids.

5. Composition according to one of Claims 1 to 4, characterised in that the anionic surface-active agent(s) is (or are) taken from the group consisting of the compounds having the following formulae:
CH₃(CH₂)₁₀CH₂OSO₃NH₄ (l)
[CH₃(CH₂)₁₀CH₂(OCH₂CH₂)ₙOSO₃]₂Mg (m)
CH₃(CH₂)₁₀CH₂OSO₃H.H₂NCH₂CH₂OH (n) 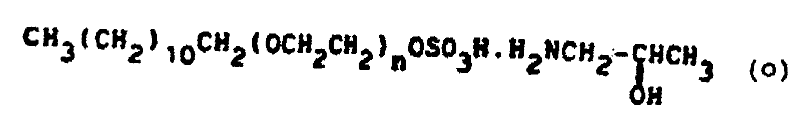
CH₃(CH₂)₁₀CH₂OSO₃Na (p)
CH₃(CH₂)₁₂CH₂(OCH₂CH₂)ₙOSO₃Na (q)
CH₃(CH₂)₁₀CH₂(OCH₂CH₂)ₙOSO₃H.N (CH₂CH₂OH)₃ (r)
CH₃(CH₂)₁₀CH₂(OCH₂CH₂)ₙO CH₂COONa (s)
C₁₃H₂₇(OCH₂CH₂)ₙOSO₃Na (t)
n, in formulae (m) to (t), being from 1 to 4.

6. Composition according to one of Claims 1 to 5, characterised in that it has a pH of between 7 and 8.5.

7. Composition according to Claim 6, characterised in that it has a pH of between 7 and 8.

8. Composition according to one of Claims 1 to 7, characterised in that it contains at least one preserving agent taken from the group consisting of sodium ethylmercurythiosalicylate, a chlorohexidine salt such as the digluconate, diacecate and dihydrochloride, a phenylmercury salt such as phenylmercury nitrate, a mixture consisting of 30% by weight of sodium benzoate and of 70% by weight of monochloroacetamide, a compound of formula (XII): 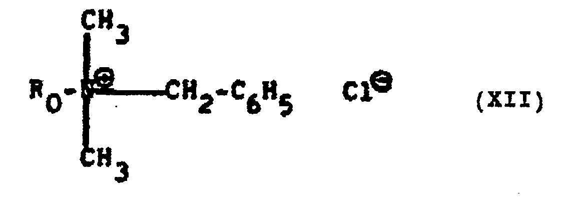 in which:
R₀ denotes a C₁₂-C₁₈ alkyl radical or a mixture of such alkyl radicals, and the C₁₂-C₁₄ and C₁₄-C₁₆ mixtures.

9. Composition according to Claim 8, characterised in that the concentration of preserving agent(s) is between 0.002 and 0.8% by weight, preferably between 0.02 and 0.5% by weight, relative to the total weight of the composition.

10. Composition according to one of Claims 1 to 9, characterised in that it contains at least one additive taken from the group consisting of moistening agents, softening agents, perfumes and colorants.

## Patentansprüche

1. Kosmetisches Reinigungsmittel zum Abschminken der Augen, mit verringerter Augenreizwirkung, enthaltend in wäßriger Lösung ein amphotheres oberflächenaktives Mittel, ein anionisches oberflächenaktives Mittel und ein nichtionisches und/oder anionisches Polymer;
dadurch gekennzeichnet,
daß es gegebenenfalls in Kombination mit einem zum Abschminken der Augen geeigneten Zusatz enthält:
1. 0,05 bis 3 Gew.-% aktives Material aus mindestens einem amphoteren oberflächenaktiven Mittel, ausgewählt unter:
* Verbindungen der Formel (I): 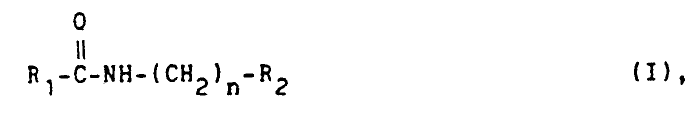 worin:
- n zwischen 1 und 3 liegt;
- R₁ eine Fettkette mit 7 bis 17 Kohlenstoffatomen darstellt ; und
- R₂ einen Rest der Formel (II) oder (III) darstellt: 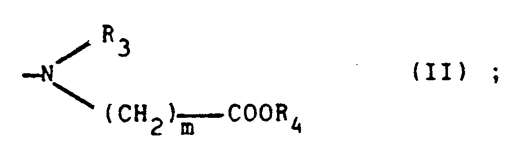 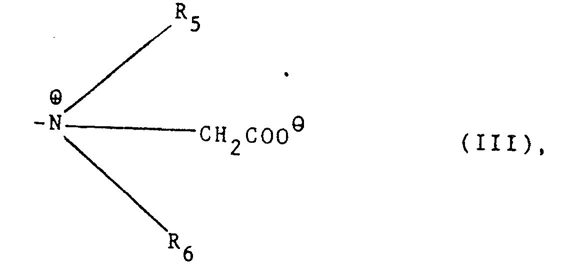 worin:
- m für 1 oder 2 steht;
- R₃ einen Rest -CH₂CH₂OR₄ oder einen Rest -CH₂CH₂OCH₂CH₂COOR₄ darstellt;
- R₄ ein Wasserstoffatom oder ein Alkalimetall bedeutet;
- R₅ einen C₁-C₂-Alkyl- oder Hydroxyalkylrest darstellt; und
- R₆ einen C₁-C₂-Alkyl- oder Hydroxyalkylrest oder einen -(CH₂)ₘCOOR₄ Rest bedeutet, wobei m und R₄ die zuvor angegebenen Bedeutungen besitzen;
* Verbindungen der Formel (IV) : 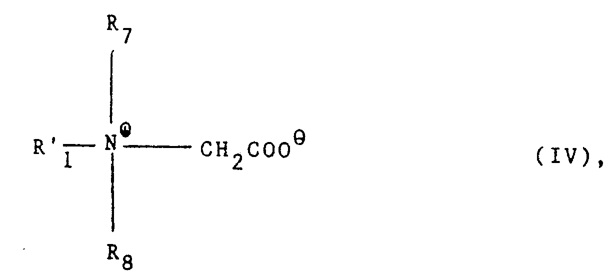 worin:
R'₁ eine Fettkette mit 8 bis 18 Kohlenstoffatomen bedeutet;
R₇ und R₈ unabhängig voneinander einen C₁-C₂-Alkylrest bedeuten; und
* Verbindungen der Formeln (V), (VI) oder (VII): 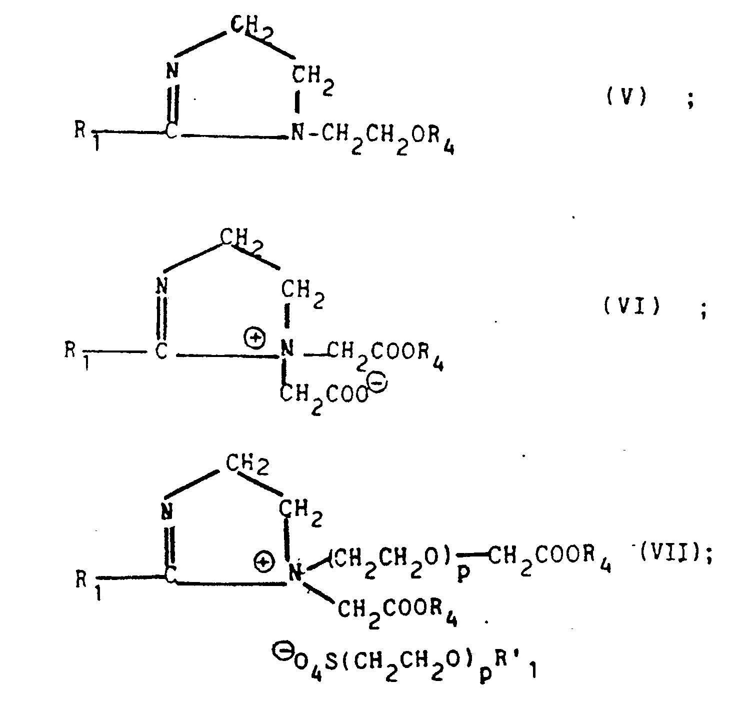 worin
- R₁, R'₁ und R₄ die oben angegebenen Bedeutungen besitzen; und
- p zwischen 0 und 4 liegt;
2. 0,05 bis 3 Gew.-% aktives Material an mindestens einem anionischen oberflächenaktiven Mittel, ausgewählt unter Verbindungen der Formel (VIII), (IX), (X) oder (XI): 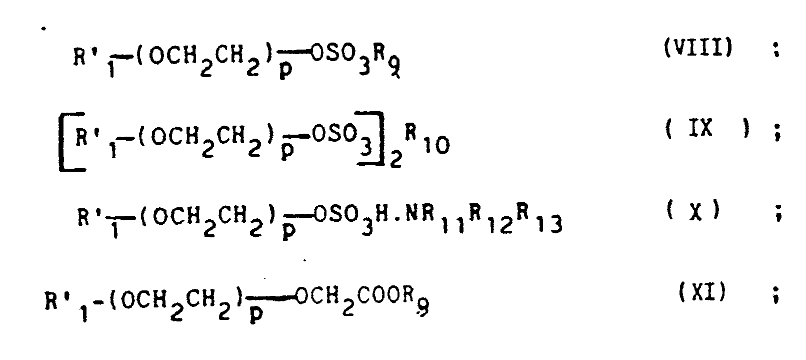 worin:
- R'₁ und p die zuvor angegebenen Bedeutungen besitzen;
- R₉ ein Alkalimetall darstellt;
- R₁₀ ein Erdalkalimetall bedeutet;
- R₁₁, R₁₂ und R₁₃ unabhängig voneinander Wasserstoff, einen C₁-C₃ Alkylrest oder einen C₁-C₃ Hydroxyalkylrest darstellen;
3. 0,02 bis 8 Gew.-% mindestens eines nicht-ionischen Polymeren, ausgewählt unter Poly-ß-alaninen, (C₁-C₃)-Alkylcellulosen, Polyhydroxy(C₁-C₃)-alkyl-cellulosen, Polyvinylpyrrolidon, nicht-ionischen Derivaten der Stärke und hydroxypropylierten Derivaten von Guar-Gummi und/oder mindestens einem anionischen Polymeren, ausgewählt unter Polyacrylamiden mit Carboxylatgruppen, Polyacrylamidosulfonsäure und ihren Salzen, sowie Polymeren und Homopolymeren der Acrylsäure oder Methacrylsäure und deren Salzen, mit einem Molekulargewicht kleiner 100 000,
wobei die Gesamtkonzentration an oberflächenaktiven Mitteln im Mittel 5 Gew.-% nicht übersteigt, und die obigen Prozentangaben bezogen auf das Gesamtgewicht des Mittels bezogen sind.

2. Mittel nach Anspruch 1, dadurch gekennzeichnet, daß es enthält:
* 0,1 bis 2 Gew.-% amphotere(s) oberflächenaktive(s) Mittel;
* 0,1 bis 2 Gew.-% anionische(s) oberflächenaktive(s) Mittel;
* 0,05 bis 5 Gew.-% nicht-ionische(s) und/oder anionische(s) Polymere(s).

3. Mittel nach einem der Ansprüche 1 und 2,
dadurch gekennzeichnet, daß in den im Anspruch 1 angegebenen Formeln (I), (V) und (VI) der Rest R₁ für eine C₁₁-C₁₃ Fettkette steht und daß in den ebenfalls im Anspruch 1 angegebenen Formeln (IV) und (VIII) bis (XI) der Rest R'₁ eine C₁₂-C₁₄ Fettkette darstellt.

4. Mittel nach einem der Ansprüche 1 bis 3,
dadurch gekennzeichnet, daß das (oder die) amphotere(n) oberflächenaktive(n) Mittel ausgewählt sind unter den folgenden Verbindungen: 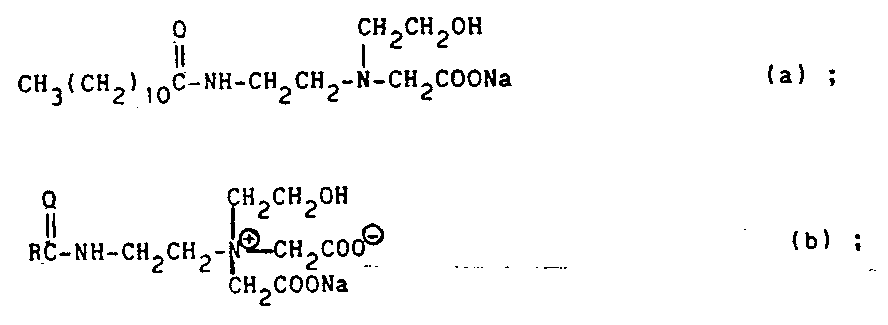 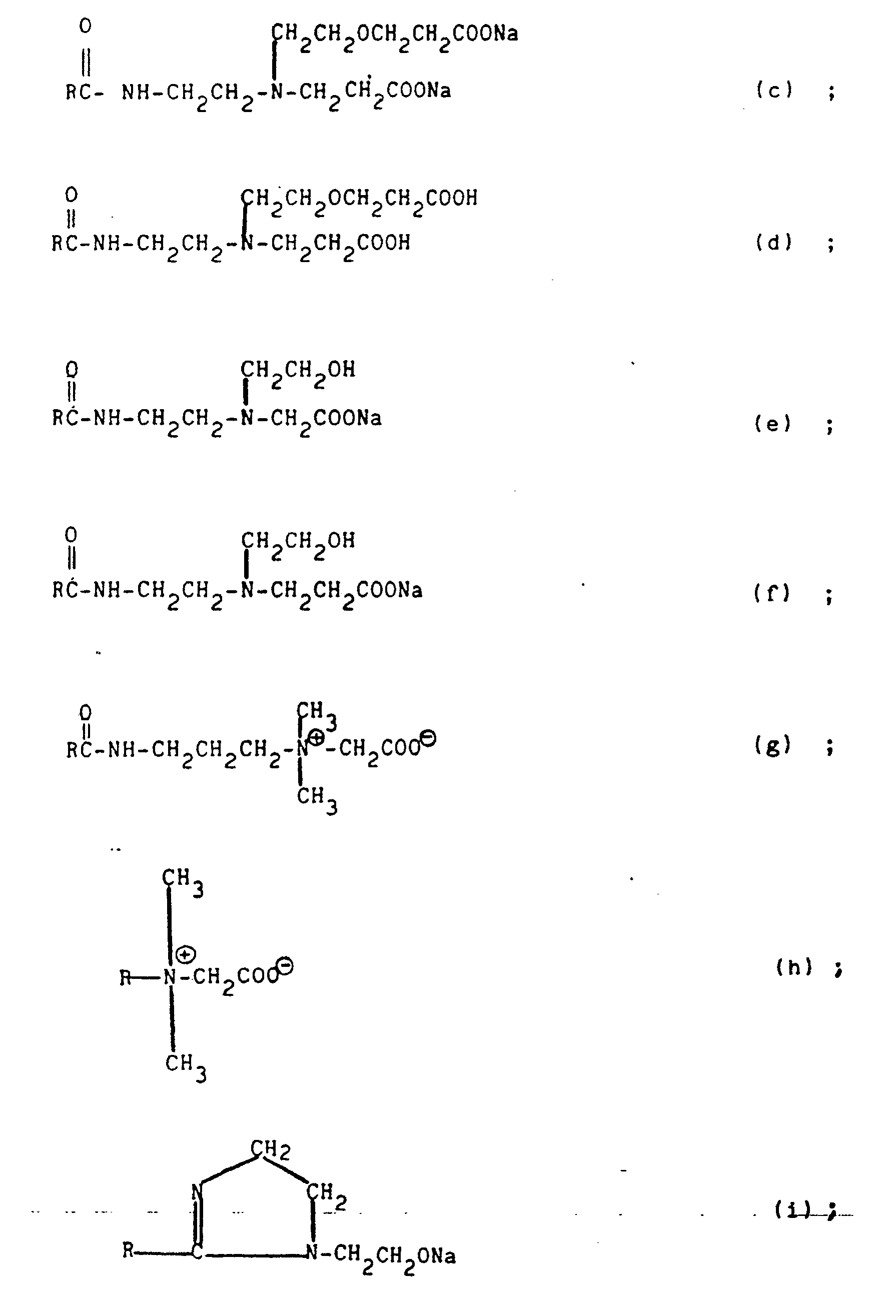 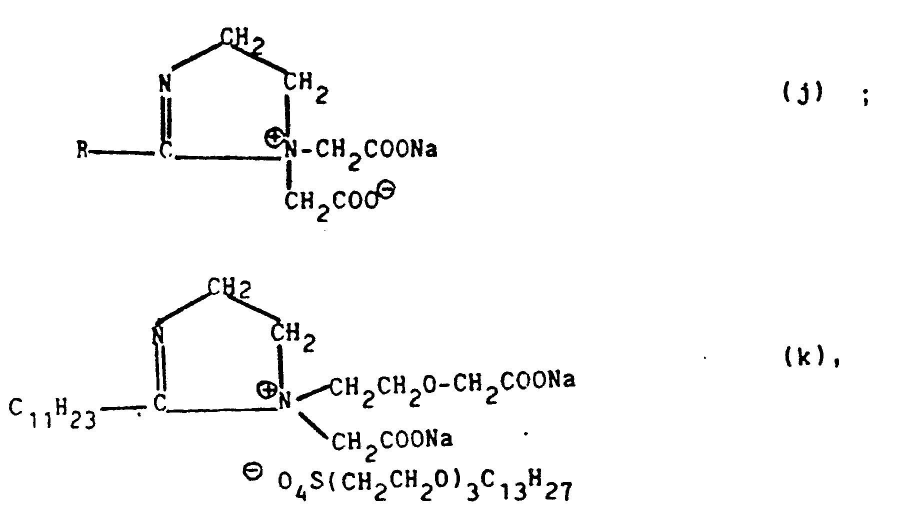 wobei in den Formeln (b) bis (j) der Rest R einen Coprafettsäurenrest darstellt.

5. Mittel nach einem der Ansprüche 1 bis 4, dadurch gekennzeichnet, daß das (oder die) anionische(n) oberflächenaktive(n) Mittel ausgewählt sind unter den Verbindungen der nachfolgenden Formel:
CH₃(CH₂)₁₀CH₂OSO₃NH₄ (l) ;
[CH₃(CH₂)₁₀CH₂(OCH₂CH₂)ₙOSO₃]₂Mg (m) ;
CH₃(CH₂)₁₀CH₂OSO₃H.H₂NCH₂CH₂OH (n) ; 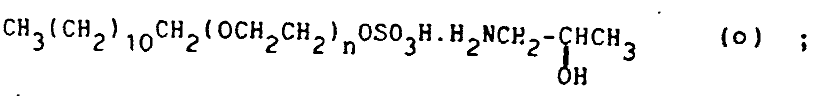
CH₃(CH₂)₁₀CH₂OSO₃Na (p) ;
CH₃(CH₂)₁₂CH₂(OCH₂CH₂)ₙOSO₃Na (q) ;
CH₃(CH₂)₁₀CH₂(OCH₂CH₂)ₙOSO₃H.N (CH₂CH₂OH)₃ (r) ;
CH₃(CH₂)₁₀CH₂(OCH₂CH₂)ₙO CH₂COONa (s) ;
C₁₃H₂₇(OCH₂CH₂)ₙOSO₃Na (t) ;
worin in den Formeln (m) bis (t) die Zahl n für 1 bis 4 steht.

6. Mittel nach einem der Ansprüche 1 bis 5, dadurch gekennzeichnet, daß es einen pH zwischen 7 und 8,5 aufweist.

7. Mittel nach Anspruch 6, dadurch gekennzeichnet, daß es einen pH zwischen 7 und 8 aufweist.

8. Mittel nach einem der Ansprüche 1 bis 7, dadurch gekennzeichnet, daß es mindestens ein Konservierungsmittel enthält, ausgewählt unter Natriumethylmercurithiosalicylat, einem Salz des Chlorhexidins, insbesondere dem Digluconat, Diacetat und Dihydrochlorid, einem Salz des Phenylquecksilbers, insbesondere Phenylquecksilbernitrat, einer Mischung, bestehend aus 30 Gew.-% Natriumbenzoat und 70 Gew.-% Monochloracetamid, einer Verbindung der Formel (XII): 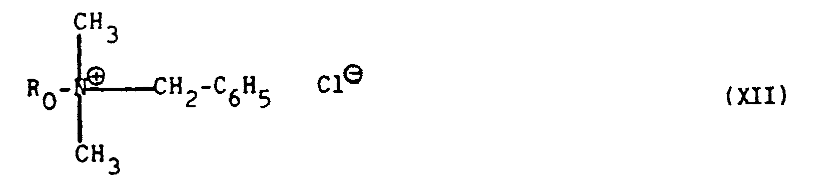 worin:
- R₀ für einen C₁₂-C₁₈ Alkylrest oder eine Mischung derartiger Alkylreste, sowie für Mischungen aus C₁₂-C₁₄ und C₁₄-C₁₆ Alkylresten, stehen.

9. Mittel nach Anspruch 8, dadurch gekennzeichnet, daß die Konzentration an Konservierungsmittel(n) zwischen 0,002 und 0,8 Gew. -%, vorzugsweise zwischen 0,02 und 0,5 Gew.-%, bezogen auf das Gesamtgewicht des Mittels, liegt.

10. Mittel nach einem der Ansprüche 1 bis 9, dadurch gekennzeichnet, daß es mindestens einen Zusatz, ausgewählt unter Befeuchtungsmitteln, Weichmachern, Parfums und Farbstoffen, enthält.
